# EUROPEAN PATENT APPLICATION

(11) **EP 4 059 572 A1**
(43) Date of publication of application: **21.09.2022**
(21) Application number: 20888103.7
(22) Date of filing: 12.11.2020
(51) Int. Cl.: A61P 43/00, C12N 5/071, A61K 35/12

(54) **CELL REPROGRAMMING METHOD**

(30) Priority: 14.11.2019 JP 2019206270
(71) Applicant: Shimasaki, Takeo, Kanazawa-shi, Ishikawa 921-8034 (JP)
(72) Inventor: YAMAMOTO, Satoko, Kahoku-gun, Ishikawa 920-0293 (JP); TOMOSUGI, Naohisa, Kahoku-gun, Ishikawa 920-0293 (JP); SHIMASAKI, Takeo, Kanazawa-shi, Ishikawa 921-8034 (JP)
(74) Representative: van Dam, Vincent
(86) International application number: PCT/JP2020/043095
(87) International publication number: WO 2021/095887

(57) **Abstract**

The present invention pertains to a method for creating reprogramed cells from somatic cells without gene introduction. The method includes a step (a) for culturing somatic cells in a medium containing a histone deacetylase inhibitor, and a step (b) for culturing the cells cultured in step (a) in a medium containing an OCT3/4 transcription stimulating factor to create reprogrammed cells.

## Description

### TECHNICAL FIELD

### Related Application:

This application claims priority to Japanese Patent Application No. 2019-206270 filed on November 14, 2019, the contents of which are incorporated herein by reference.

### Technical Field:

The present invention pertains to a method for reprograming cells, and more specifically pertains to a method for reprograming cells using a chemical substance without gene introduction from outside.

### BACKGROUND ART

Since embryonic stem cells (ES cells) were established from a mouse embryo in 1981 (Non-Patent Document 1), the ES cells have been widely used as one material for tissue regeneration studies up to the present. Multipotent/pluripotent stem cells, such as ES cells, feature multipotency/pluripotency, and the use of this property is considered to ensure regenerating various kinds of tissues. The ES cells are expected to be used for treatment of diseases that have been difficult to be completely cured up to the present, specifically, a neurodegenerative disease such as Parkinson disease, spinal cord injury, cerebral infarction, diabetes, cirrhosis, and cardiomyopathy.

However, because of transplantation of ES cells or a tissue regenerated from ES cells being allotransplant, a problem arises in that rejection after transplantation is evoked similarly to organ transplantation. Additionally, for establishment of ES cells, a fertilized egg and an early embryo at a phase up to a blastocyst where generation progresses from a fertilized egg are required. Especially, in the case of a human, the use of a fertilized egg of a human as a material loses a birth of life, and therefore a problem from an ethical viewpoint has also been pointed out.

On the other hand, in 2006, Yamanaka, et al. reported the breakthrough creation technique of artificial multipotent/pluripotent stem cells (iPS cells), which establishes multipotent/pluripotent cells through introduction of four genes (an OCT3/4 gene, a Sox gene, a c-Myc gene, and a Klf gene) into somatic cells (Non-Patent Document 2). The iPS cells allow autotransplantation by use of somatic cells, and further eliminate the need for embryo break, which is ethically problematic, thus solving the problem in the ES cells. Thus, the dramatic advancement in regeneration medicine technique with the iPS cells is common knowledge. Establishment of human iPS cells succeeded in 2007 (Non-Patent Documents 3 and 4), and many reports regarding the iPS cell have been made up to the present.

Nowadays, the establishment method of iPS cells seems to dramatically advance from the beginning of the discovery of iPS cells. A retrovirus, which had been used for gene introduction at an initial phase since reported first, was controversial in a possibility of carcinogenesis due to insertion mutation to a somatic cell genome. However, currently, a method that does not introduce a mutation into a somatic cell genome, such as a method with an episomal vector and a synthetic mRNA, in addition to a Sendai virus as an RNA virus, has been developed (Non-Patent Documents 5 to 8). Additionally, kinds of genes to be introduced have also been variously improved, such as development of a method of eliminating the need for genes other than an OCT3/4 gene or a Sox gene (Non-Patent Document 9).

In addition to the iPS cells, multipotent/pluripotent stem cells referred to as Muse cells have been reported (Patent Document 1). The Muse cells are multipotent/pluripotent stem cells not having a neoplasm present in a biological tissue, such as bone marrow and skin, and distinctive in being positive in stage specific embryonic antigen-3 (SSEA-3). Differentiation of Muse cells into various cells of all of three germ layers, such as a hepatocyte, a muscle, a nerve, a glia cell, a pigment cell of skin (melanocyte), a cuticle, and a blood vessel, has been reported up to the present. Thus, the concern for the multipotent/pluripotent stem cells in regeneration medicine is high, and further establishment of the technique has been desired for creation of multipotent/pluripotent stem cells.

Patent Document 1: WO2012/133948

Non-Patent Document 1: Martin GR, Proc Natl Acad Sci U S A. 1981 Dec; 78(12):7634-8.
Non-Patent Document 2: Takahashi K et al., Cell. 2006 Aug 25; 126(4):663-76.
Non-Patent Document 3: Takahashi K et al., Cell. 2007 Nov 30; 131(5):861-72.
Non-Patent Document 4: Yu J et al., Science. 2007 Dec 21; 318(5858):1917-20.
Non-Patent Document 5: Okita K et al., Nat Methods. 2011 May; 8(5):409-12.
Non-Patent Document 6: Agu CA et al., Stem Cell Reports. 2015 Oct 13; 5(4):660-71.
Non-Patent Document 7: Warren L et al., Cell Stem Cell. 2010 Nov 5; 7(5):618-30.
Non-Patent Document 8: Yakubov E et al., Biochem Biophys Res Commun. 2010 Mar 26; 394(1):189-93.
Non-Patent Document 9: Shi Y et al., Cell Stem Cell. 2008 Jun 5; 2(6):525-8.

### DISCLOSURE OF THE INVENTION

### PROBLEMS TO BE SOLVED BY THE INVENTION

From the aspect of clinical application, it is significantly important for creation of the multipotent/pluripotent stem cells to use somatic cells as a raw material and not to perform an artificial gene operation. Although the possibility, the cost aspect, the creation efficiency, the creation period, and the like of genetic modification have been improved up to the present, additional improvement is a problem required to be solved in the future. In consideration of the problem, an object of the present invention is to provide a method for reprograming cells, and more specifically, provide a method for reprograming cells without an introduction operation of an artificial gene from outside, as a part of a creation technique of multipotent/pluripotent stem cells.

### SOLUTIONS TO THE PROBLEMS

Through results of serious examinations to solve the problem, the inventors have found the following. After culturing somatic cells in a medium containing a histone deacetylase inhibitor (such as 2-mercaptoethanol), which is a DNA function damaging substance, when the somatic cells were cultured in a medium containing a transcription stimulating factor of OCT3/4 (such as LIF, CCL2, and IL-6), these somatic cells were reprogramed. The inventors have completed the present invention based on the knowledge.

The present invention is preferably embodied by the aspects described below, but is not limited to the aspects.
[1] A method for creating reprogramed cells from somatic cells without gene introduction includes: a step (a) of culturing the somatic cells in a medium containing a histone deacetylase inhibitor; and a step (b) of culturing the cells cultured in the step (a) in a medium containing an OCT3/4 transcription stimulating factor to create the reprogrammed cells.
[2] In the method according to [1], the OCT3/4 transcription stimulating factor is any one or more selected from the group consisting of LIF, CCL2, and IL-6.
[3] In the method according to [1] or [2], the histone deacetylase inhibitor is 2-mercaptoethanol.
[4] A method for creating reprogramed cells from somatic cells without gene introduction includes: a step (a) of culturing somatic cells in a medium containing 2-mercaptoethanol; and a step (b) of culturing the cells cultured in the step (a) in a medium containing LIF to create the reprogrammed cells.
   A culture period in the step (a) is preferably three days or more, more preferably three to seven days, a culture period in the step (b) is preferably three days or more, more preferably three to nine days, and the cells are preferably cultured until spheroid formation is confirmed.
[5] In the method according to any one of [1] to [4], the medium in the step (a) contains 10 µM to 0.2 mM, preferably 10 µM to 100 µM, and more preferably 10 µM to 50 µM of 2-mercaptoethanol.
[6] In the method according to claim [4] or [5], the medium in the step (b) further contains LIF.
   Preferably, the medium in the step (b) contains 1 to 100 ng/mL (100 to 5000 units/ml as a titer) of LIF.
[7] In the method according to any one of [1] to [6], the medium in the step (b) further contains one or more selected from ACTH, bFGF, and a GSK3β inhibitor.
[8] In the method according to any one of [1] to [7], the medium in the step (a) does not contain the OCT3/4 transcription stimulating factor. Preferably, the medium in the step (a) does not contain a cytokine or a hormone.
[9] In the method according to any one of [1] to [8], the reprogrammed cells are multipotent stem cells that are allowed to be differentiated into three germ layers. The multipotent stem cells are possibly pluripotent.
[10] In the method according to any one of [1] to [8], the somatic cells are fibroblasts and preferably skin fibroblasts. In the step (a), the cells are preferably cultured until a form change of the fibroblasts is confirmed.
[11] In the method according to any one of [1] to [8], the somatic cells are human fibroblasts, and preferably human skin fibroblasts.
[12] A kit for reprogramming somatic cells containing the following reagent A and reagent B:
   the reagent A) a reagent for constituting a medium not containing 2-mercaptoethanol; and
   the reagent B) a reagent for constituting a medium containing LIF, CCL2, and IL-6.
   The media and components for the reagent A and the reagent B may be separately packed and prepared at time of use, or the respective components may be contained in the media. Additionally, the medium containing the reagent A does not contain the OCT3/4 transcription stimulating factor (LIF, CCL2, or IL-6).
[13] In the kit according to [12], the reagent B further contains one or more selected from ACTH, bFGF, and a GSK3β inhibitor as a component.
[14] A method for producing aimed cells from somatic cells includes: a step (i) of creating the reprogrammed cells from the somatic cells by the method according to any one of claims 1 to 11; and a step (ii) of performing differentiation induction on the reprogrammed cells to the aimed cells.
[15] A method for producing a cell preparation includes: a step (i) of creating the reprogrammed cells from the somatic cells by the method according to any one of [1] to [11]; a step (ii) of performing differentiation induction on the reprogrammed cells to aimed cells; and a step (iii) of preparing a cell preparation containing a pharmacologically acceptable carrier with the differentiatedly induced cells.
[16] The cells induced from the somatic cells by the method according to [14].
[17] A cell preparation contains a pharmacologically acceptable carrier with the cells prepared by the method according to [15].

### ADVANTAGEOUS EFFECTS OF THE INVENTION

According to the present invention, the method for reprograming the cells without an introduction operation of an artificial gene from outside can be provided. The method provided by the present invention can be used to create multipotent/pluripotent stem cells. Since the method of the present invention eliminates the need for introduction of the artificial gene from the outside, a risk of affecting somatic cells, such as a problem of immunogenicity and a carcinogenic risk, can be significantly reduced low. Additionally, according to the method of the present invention, only use of an HDAC inhibitor during the culture of cells is enough, and therefore the cells can be reprogrammed easily and stably. Furthermore, costs for reprograming of the cells and creation of the multipotent/pluripotent stem cells can be reduced low. From these aspects, the use of the method of the present invention allows further facilitating studies on multipotent/pluripotent stem cells, thereby bringing a significant contribution to study and development of tissue regeneration technique in the future.

### BRIEF DESCRIPTION OF THE DRAWINGS

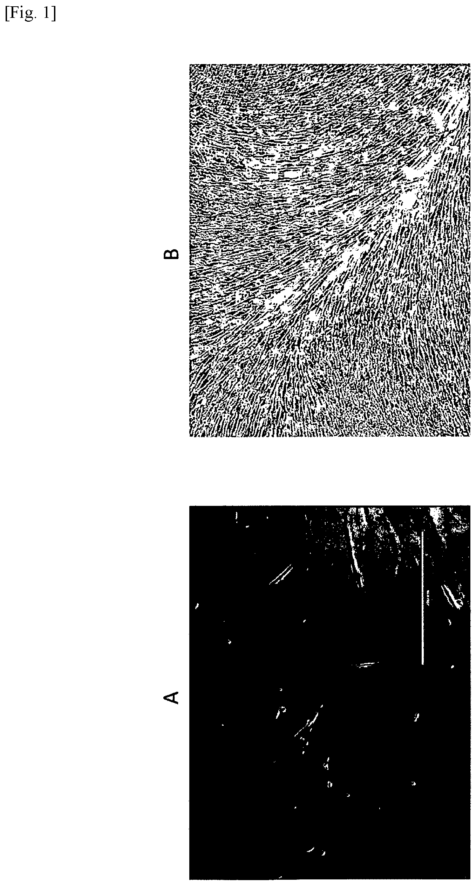

Fig. 1 includes drawings illustrating a form of cells after culturing human skin fibroblasts. Fig. 1A illustrates the form of the human skin fibroblasts in a usual state (cell density: small) after the culture. Fig. 1B is a drawing illustrating the human skin fibroblasts in a high cell density state after the culture.

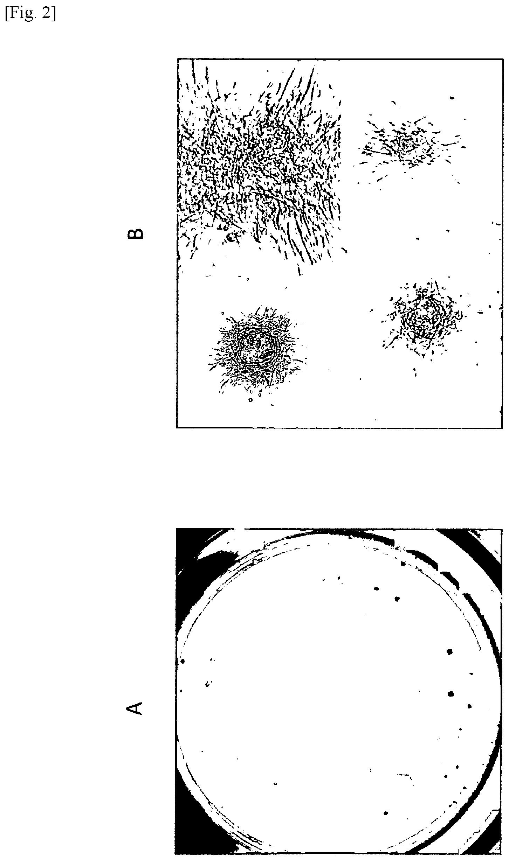

Fig. 2 includes drawings illustrating spheroids formed after culturing the human skin fibroblasts. Fig. 2A is a photograph in which the spheroids formed after culturing the human skin fibroblasts are slightly enlarged, and Fig. 2B is a photograph in which the spheroids are further enlarged.

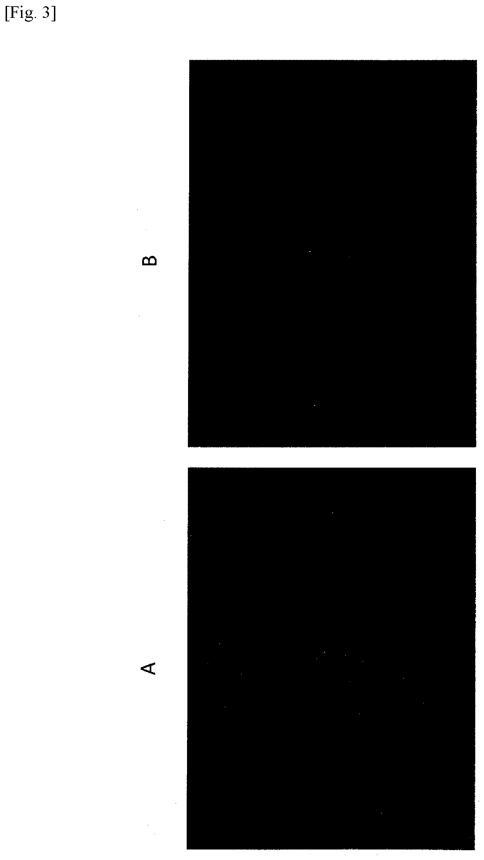

Fig. 3 includes drawings showing an alkaline phosphatase reaction of the cells after culture.

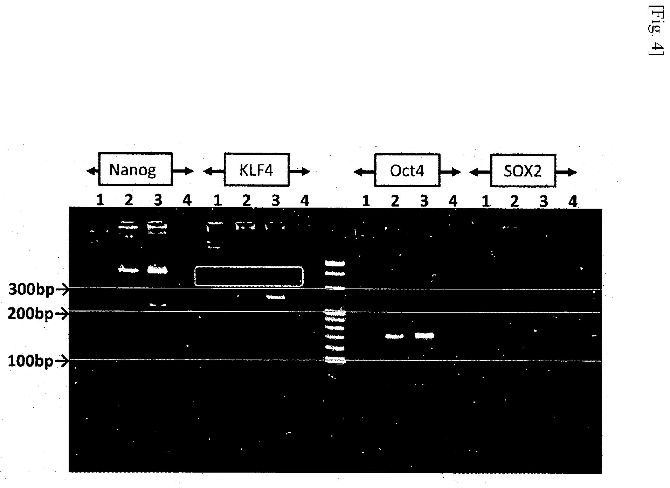

Fig. 4 is a drawing illustrating results of PCR experiment that examined expression of undifferentiated markers of Nanog, Klf4, Oct4, and Sox2. Planned sizes of Nanog, Klf4, Oct4, and Sox2 are 406 pb, 396 bp, 143 bp, and 150 bp, respectively. A lane 1 shows a human skin fibroblast sample on which a process is not performed with 2-mercaptoethanol, a lane 2 shows a human skin fibroblast sample on which a process is not performed with 2-mercaptoethanol and culture was performed by an incubator performing a step (a), a lane 3 shows a sample produced by performing a process on human skin fibroblasts with 2-mercaptoethanol and reprogramming the human skin fibroblasts, and a lane 4 shows a negative contrast sample to which water was added instead of RNA.

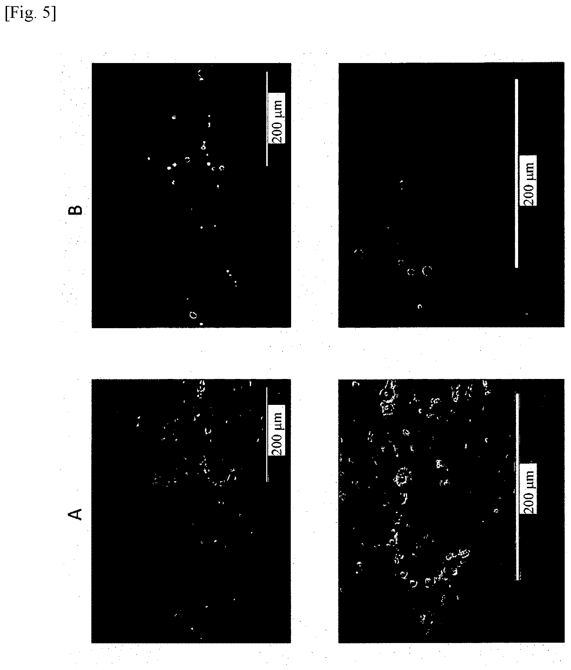

Fig. 5 includes drawings illustrating results of differentiation into adipose cells. Fig. 5A is an enlarged photograph after the differentiation into the adipose cells, and Fig. 5B is a photograph of performing fluorescence stain on the adipose cells after differentiation.

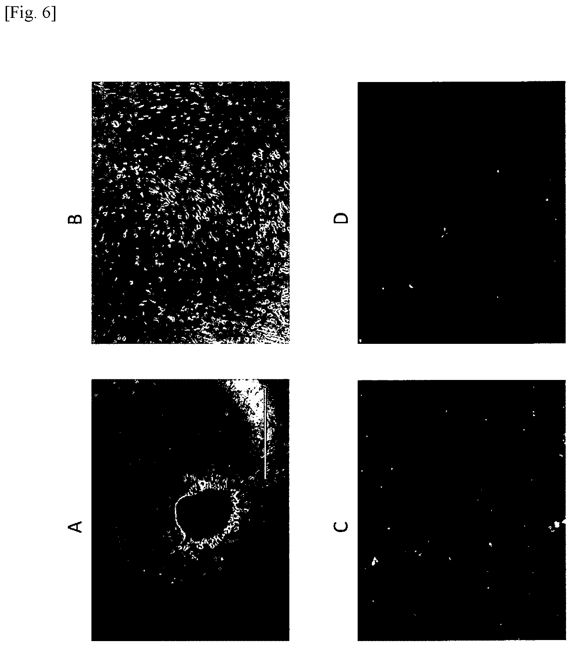

Fig. 6 includes drawings illustrating results of differentiation into nerve cells. Figs. 6A to 6C are photographs after the differentiation into the nerve cells, and Fig. 6D is a photograph of performing immunostaining on the nerve cells after differentiation using an antibody (anti-neurofilament antibody).

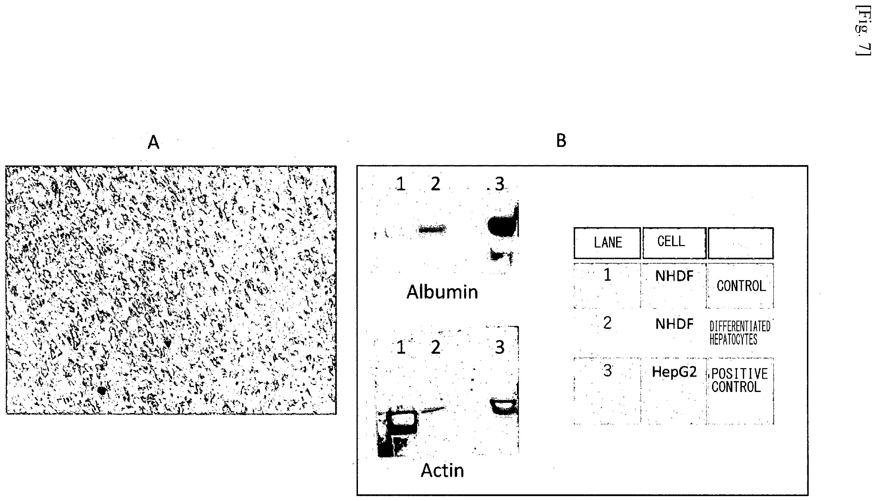

Fig. 7 includes drawings illustrating results of differentiation into hepatocytes. Fig. 7A illustrates that the hepatocytes after differentiation are PAS-stained, and Fig. 7B illustrates that the differentiated hepatocytes produce albumin.

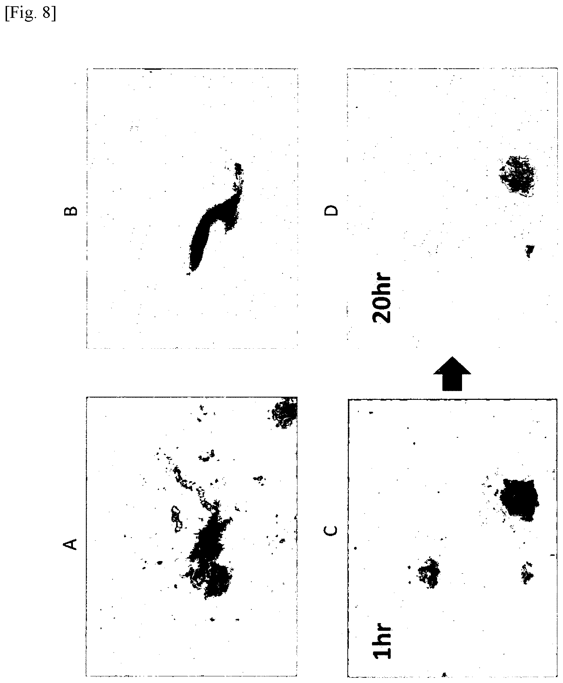

Fig. 8 includes drawings illustrating results regarding the hepatocytes after differentiation. Fig. 8A shows that indocyanine green (ICG) is taken in the hepatocytes after differentiation. Fig. 8B shows that the hepatocytes after differentiation exhibit a bile duct-like structure and the ICG is accumulated in the bile duct-like structure. Fig. 8C is a photograph after one hour from taking in the ICG, and Fig. 8D shows that the ICG almost disappeared after 20 hours.

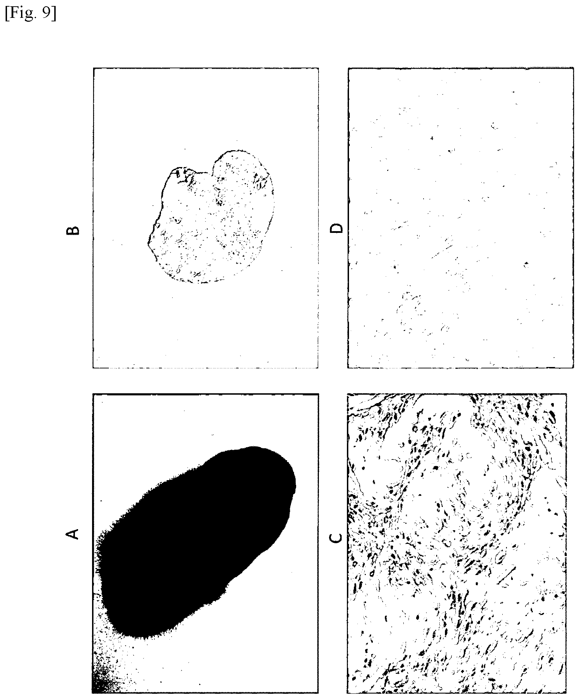

Fig. 9 includes drawings illustrating results of differentiation into cartilage cells. Fig. 9A is a photograph of the differentiated cartilage cells. Fig. 9B is a HE stain photograph of the differentiated cartilage cells. Fig. 9C is an enlarged photograph of Fig. 9B. Fig. 9D is a photograph after the cartilage cells after differentiation are stained with alcian blue.

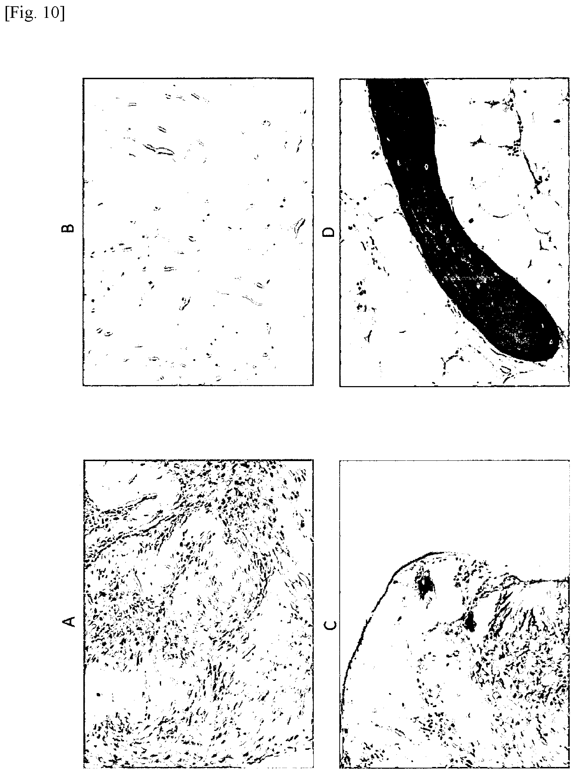

Fig. 10 includes drawings illustrating results regarding the cartilage cells after differentiation. Fig. 10A is a photograph of the enlarged cartilage cells after differentiation, and Fig. 10B is a photograph illustrating a fibrocartilage of an articular labrum of a human. Fig. 10A and Fig. 10B illustrate that the cartilage cells after differentiation and the fibrocartilage of the articular labrum of the human mutually have similar forms pathologically. Fig. 10C illustrates that a part of the cartilage cells after differentiation is ossified. Fig. 10D is a photograph of bone tissue of a caput of bone of a human. Fig. 10C and Fig. 10D show that the ossified part of the cartilage cells after differentiation has a form similar to the bone tissue of the human pathologically.

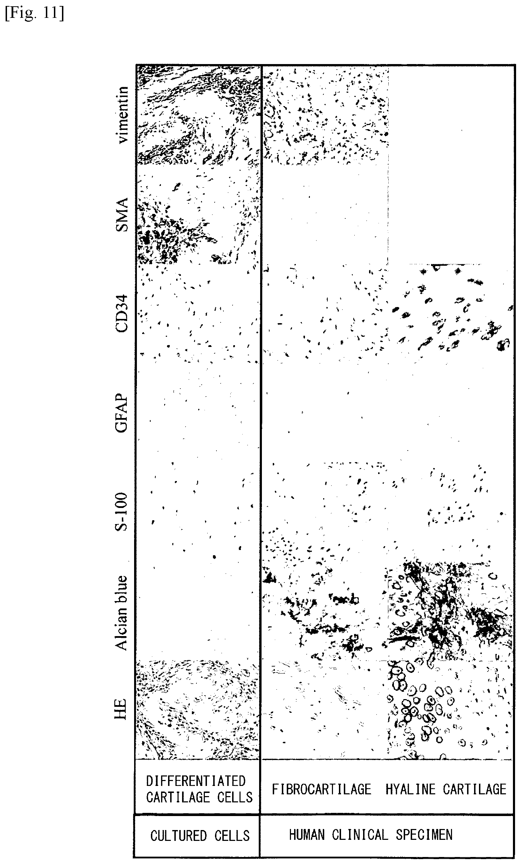

Fig. 11 is a drawing illustrating results of cartilage cells after differentiation and cartilage cells in a human clinical specimen are stained with various kinds of antibodies and a medicinal solution. Due to their biological features, the cartilage cells after differentiation are remarkably similar to the fibrocartilage in the human clinical specimen.

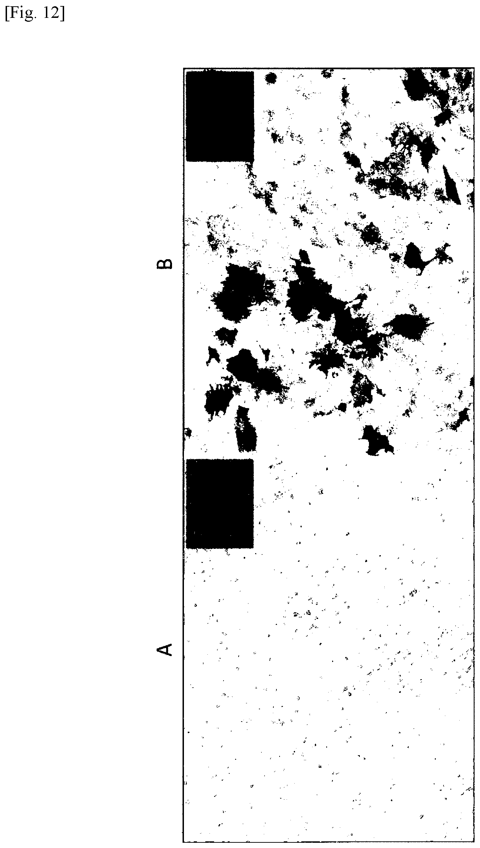

Fig. 12 is a drawing illustrating a result of staining osteoblasts after differentiation with alkaline phosphatase. Fig. 12A illustrates controls (skin fibroblasts), and Fig. 12B illustrates cells produced by performing differentiation induction on the cells after reprogramming to osteoblasts. The cells on which the differentiation induction is performed are positive in alkaline phosphatase.

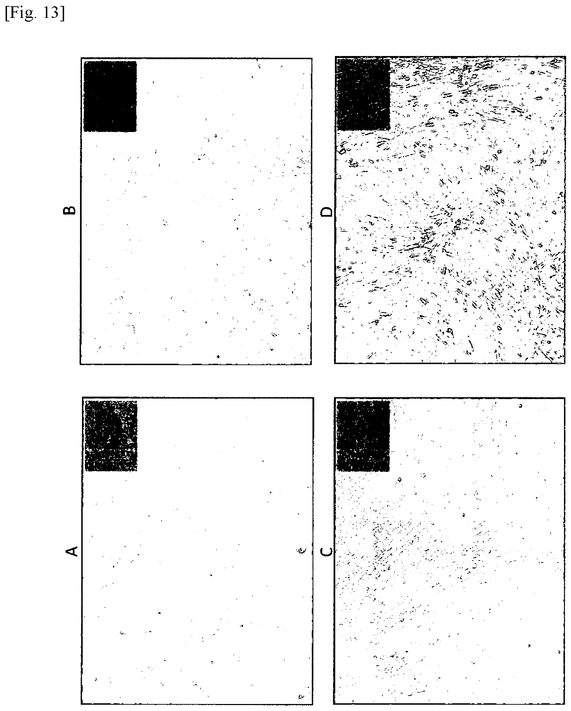

Fig. 13 includes drawings illustrating forms of cells after culturing human skin fibroblasts cultured in media containing LIF/CCL2 after culture in D-MEM media containing 2-mercaptoethanol. Fig. 13A illustrates the usual fibroblasts, Fig. 13B illustrates the fibroblasts using only the LIF, Fig. 13C illustrates the fibroblasts using LIF + CCL2 (10 ng/mL), and Fig. 13D illustrates the fibroblasts using LIF + CCL2 (100 ng/mL).

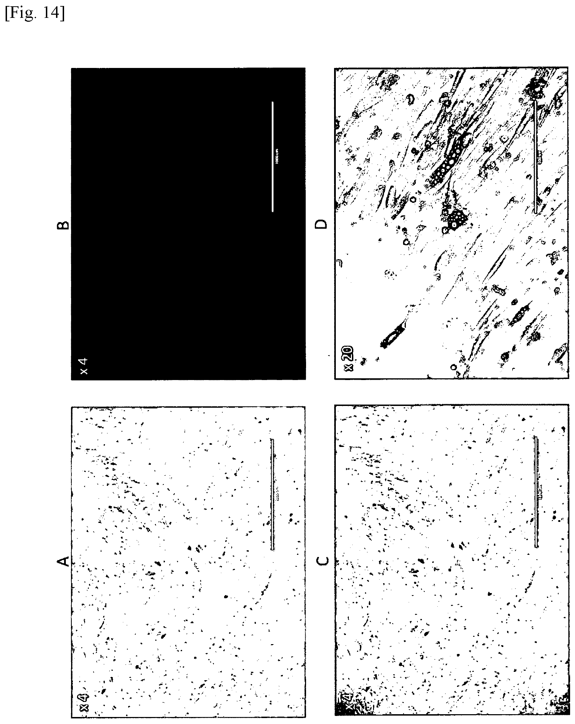

Fig. 14 illustrates differentiation induction to adipose cells. Fig. 14A illustrates cells after culture for seven days in a D-MEM medium containing 2-mercaptoethanol, Fig. 14B illustrates cells after culture for seven days in a medium containing LIF + CCL2 (10 ng/mL), and Figs. 14C and 14D illustrate cells on the 25th day after differentiation induction to the adipose cells (4 powers and 20 powers, respectively).

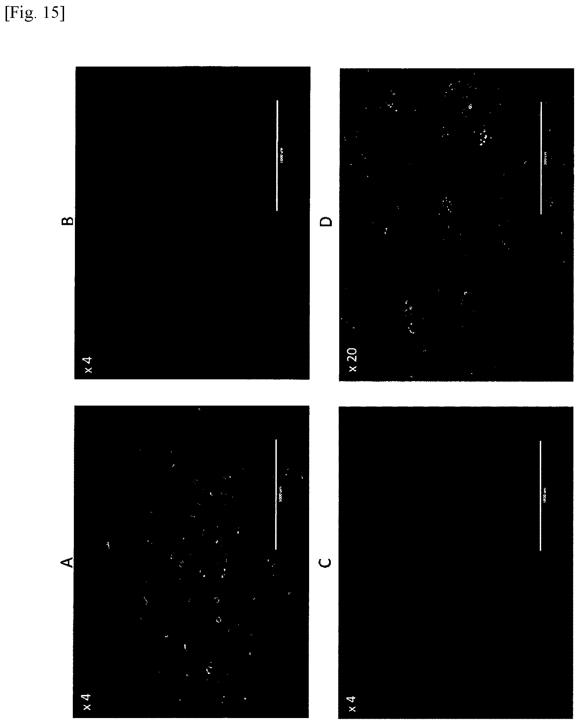

Fig. 15 illustrates differentiation induction to adipose cells. Fig. 15A illustrates cells after culture for seven days in a D-MEM medium containing 2-mercaptoethanol, Fig. 15B illustrates cells after culture for seven days in a medium containing LIF + CCL2 (100 ng/mL), and Figs. 15C and 15D illustrate cells on the 25th day after differentiation induction to the adipose cells (4 powers and 20 powers, respectively).

### DESCRIPTION OF PREFERRED EMBODIMENTS

One aspect of the present invention is a method for reprogramming cells that includes a step of culturing cells in a medium containing a histone deacetylase inhibitor (such as 2-mercaptoethanol) and a step of culturing the cells in a medium containing an OCT3/4 transcription stimulating factor (such as LIF, CCL2, and IL-6).

In this Description, the reprogramming means returning differentiated cells into multipotent/pluripotent stem cells again, and the reprogramming of cells is also referred to as initialization of cells. Biologically, reprogramming means deletion or restructure of an epigenetic label, such as DNA methylation. A representative example of reprograming of cells includes an iPS cell generation technique that introduces an initialization factor into somatic cells. In the present invention, reprogramming is performed using an HDAC inhibitor, which is a DNA function damaging substance, without gene introduction. Note that in this Description, a step of culturing cells in a medium containing the DNA function damaging substance (HDAC inhibitor) is referred to as a step (a) below.

### <DNA Function Damaging Substance>

The DNA function damaging substance means a substance that bonds to deoxyribonucleic acid (DNA) or directly or indirectly acts on DNA to ensure damaging any of DNA synthesis, DNA transcription (RNA synthesis), and DNA replication, which are functions that the DNA has.

### <Histone Deacetylase Inhibitor>

The present invention uses a histone deacetylase inhibitor as the DNA function damaging substance. Histone deacetylase (HDAC) is an enzyme that deacetylates histone as a main constituent factor in a chromatin structure. The HDAC inhibitor inhibits this HDAC to control transcription of a gene. In further detail, while the histone is present in an aggregated state in cells in a differentiated state, the HDAC inhibitor relaxes the aggregated chromatin structure to allow the stopped transcription of an initialization-related gene to start.

Examples of the HDAC inhibitor used in the present invention include 2-mercaptoethanol, ethylene oxide, butyric acid, apicidin, valproic acid, trichostatin A, and vorinostat.

2-mercaptoethanol is a compound expressed by a structural formula HS-CH₂-CH₂-OH and also referred to as β-mercaptoethanol or thioglycol. The CAS registry number of 2-mercaptoethanol is 60-24-2. 2-mercaptoethanol can be synthesized by reaction between hydrogen sulfide and ethylene oxide, and as the 2-mercaptoethanol, an industrially manufactured commercial product can be preferably used. For example, a reagent and a product of FUJIFILM Wako Pure Chemical can be used.

Ethylene oxide is a compound expressed by molecular formula C₂H₄O, also referred to as epoxyethane, oxirane, oxasilacyclopropane, oxidized ethylene, and ethylene oxide, and abbreviated as EO. The CAS registry number of ethylene oxide is 75-21-8. Ethylene oxide can be synthesized by reaction between ethylene and oxygen, and as the ethylene oxide, an industrially manufactured commercial product can be preferably used. For example, the product of Mitsubishi Chemical Corporation, Showa Denko Gas Products, and Taiyo Nippon Sanso can be used. With the use of ethylene oxide, the ethylene oxide may be contained in a medium of cells, but the ethylene oxide is preferably used by spraying an ethylene oxide gas to a cell culture tool (such as a culture container).

In the present invention, in addition to the above-described 2-mercaptoethanol and ethylene oxide, other HDAC inhibitors can be used similarly to these compounds, but the use of 2-mercaptoethanol is preferred.

### <Cells>

The cells cultured by the method of the present invention are preferably somatic cells. As long as cells in which differentiation is in progress, the somatic cells in the present invention are not especially limited, and any cells other than reproductive cells or cell masses thereof are usable. Example of the kinds of somatic cells include already differentiated cells, such as fibroblasts, adipose cells, nerve cells, muscle cells (such as cardiac muscle cells, smooth muscle cells, and skeletal muscle cells), skin cells, epithelial cells, endothelial cells, blood cells (such as neutrophil, eosinophil, basophil, monocyte, and lymphocyte), hepatocytes, nephrocytes, lung cells, pancreatic cells, hair matrix cells (such as skin hair matrix cells and body hair matrix cells), and buccal cells (such as oral mucosal cells), and haematopoietic stem cells, mesenchymal stem cells, neural stem cells, and somatic stem cells, such as adipose-derived stem cells, or various kinds of precursor cells, but the somatic cells are not limited to them. In the present invention, from the aspect of ease of obtaining the cells, fibroblasts (more preferably, skin fibroblasts), buccal cells, and hair matrix cells are preferred, fibroblasts are more preferred, and skin fibroblasts are especially preferred.

The somatic cells can be extracted from animals, such as mammals and birds. Examples of the mammals (mammalian) include primates, such as a human, a monkey, a chimpanzee, a gorilla, and an orangutan, rodents, such as a mouse and a rat, and a rabbit, a dog, a cat, cattle, a pig, a goat, a sheep, and a horse, and examples of birds include a chicken and a duck. The mammals and the birds are not limited to them. The somatic cells may be somatic cells in a fetal period or may be matured somatic cells. The somatic cells may be primary-cultured cells or may be passage cells. To transplant the obtained reprogramed cells or the differentiated cells or tissue thereof, the use of somatic cells (that is, own somatic cells) extracted from an animal target for transplantation or somatic cells extracted from an animal same kind of this animal is preferred. Alternatively, when the transplantation involves treatment of an illness, the cells are preferably the somatic cells of the tissue involved in this illness.

### <Culture of Cells>

The present invention includes the step of culturing the cells in the medium containing the HDAC inhibitor and allows reprograming the cells by at least performing this step. These cells only need to be cultured in a state in contact with the medium containing the HDAC inhibitor, and are preferably cultured in the medium containing the HDAC inhibitor.

Although the content of HDAC inhibitor in the medium is not specifically limited, for example, a rate of the number of cells after one week from when the culture starts can be expressed as an amount 70% or more (preferably 80% or more, more preferably 90% or more, and further preferably 95% or more) with respect to the number of cells when the culture is performed without containing the HDAC inhibitor for one week (when the culture is performed for one week under the identical conditions except that the HDAC inhibitor is not contained). Additionally, a part of the cells after one week from when the culture starts can be expressed as the amount indicating the form (spheroids) of the aggregations of cells. This content can be appropriately set according to the kind of the HDAC inhibitor, the culture condition, and the like, but, for example, the content is 0.01 µM to 50 mM, preferably 0.1 µM to 10 mM, more preferably 0.5 µM to 7 mM, and further preferably 1 µM to 5 mM.

With the use of 2-mercaptoethanol as the HDAC inhibitor, the content of HDAC inhibitor in the medium is, for example, 0.1 µM to 10 mM, preferably 0.1 µM to 2 mM, more preferably 0.1 µM to 5 mM, and further preferably from 10 µM to 3 mM. Considering the influence on the cells, the amount of 2-mercaptoethanol is preferably as little as possible to the extent that reprograming is possible. From the aspect, the 2-mercaptoethanol may be 10 µM to 0.2 mM, may be 10 µM to 100 µM, and 10 µM to 50 µM. Note that since the concentration of 2-mercaptoethanol usually used for culturing the stem cells is 100 µM, it can be said that the reprograming of the present invention can be performed with the 2-mercaptoethanol at the concentration much smaller than that.

As described later, in the present invention, the order that the HDAC inhibitor, such as 2-mercaptoethanol, is caused to act, and then the OCT3/4 transcription stimulating factor (such as LIF, CCL2, and IL-6) is caused to act is important for reprograming of cells. Accordingly, the medium in the step (a) does not contain the OCT3/4 transcription stimulating factor. Preferably, the medium in the step (a) does not contain any of a cytokine or a hormone, such as ACTH, bFGF, or a GSK3β inhibitor.

### <OCT3/4 Transcription Stimulating Factor>

The OCT3/4 transcription stimulating factor is a factor that stimulates the transcription of OCT3/4 for activation, and examples of which include LIF (leukemia inhibiting factor), CCL2 (chemokine (C-C motif) ligand 2), and IL-6 (interleukin 6). Among them, the use of LIF is preferred, and the use of LIF and CCL2 is more preferred.

In the present invention, the chromatin structure is relaxed with the HDAC inhibitor, and next, the OCT3/4 transcription stimulating factor is caused act to activate the transcription of OCT3/4, and the cells are transitioned to an undifferentiated state by action of protein mainly by OCT3/4.

In the method of the present invention (step (a)), conditions for culturing the cells are not especially limited. For example, a culture temperature can be 30 to 40°C, is preferably 35 to 39°C, and more preferably 36 to 38°C. Additionally, the CO₂ concentration is, for example, 1 to 10%, preferably 1.5 to 8%, and more preferably 2 to 5%.

The culture period of cells in the step (a) is also not especially limited and can be appropriately set according to the kind of used cells, the culture conditions, and the like. The culture period of cells in the present invention is, for example, 1 day or more, and the specific culture period is 1 to 10 days. Although the preferred culture period is also not especially limited, for example, the culture period is two to nine days, preferably three days or more, and more preferably three to seven days.

A medium composition used in cell culture can also be appropriately set according to the kind of used cells, the culture conditions, and the like and therefore is not specifically limited. For example, although the base medium is not specifically limited, an Eagle medium (such as BM, MEM, and DMEM), a McCoy medium (such as McCoy5A and McCoy7A), a Ham medium (such as F10 and F12), a 199 medium, a RPMI1640 medium, an NCTC medium (such as NCTC109 and NCTC135) can be used. Additionally, various kinds of base media can be mixed to be used as necessary.

To the base medium described above, an additive usually required to maintain cells, such as serum (such as fetal calf serum and human serum), serum substitute (such as KSR and B27 supplement), amino acid (such as alanine, arginine, cystine, and histidine), vitamin (such as vitamin B7 and vitamin B12), an antibiotic (such as amphotericin B and kanamycin), an adhesion factor (such as type I collagen, gelatin, and fibronectin), fatty acid (such as oleic acid, arachidonic acid, and linolenic acid), adenine, guanosine, hypoxanthine, thymidine, and cholesterol can be added.

In the present invention, as the resultant of the culture of the step (a) described above, a change in form of the cells is observed in some cases. Here, in this Description, the change in form of the cells means that the appearance of the cell mass after culture changes from the state at the time of starting the culture. For example, as illustrated in the photograph in Fig. 1B, a state in which the cells after culture start aggregating from the peripheral area is possibly observed. After confirmation of the change in form of the cells in the state, the culture step described above can be stopped.

### <Culture in Presence of OCT3/4 Transcription Stimulating Factor>

The method of the present invention further includes a step of culturing cells in a medium containing the OCT3/4 transcription stimulating factor, for example, one or more selected from the group consisting of LIF, CCL2, and IL-6 (hereinafter referred to as a step (b) in this Description), subsequent to the culture step described above.

The medium used in the step (b) may contain any one or more of an adrenocorticotropic hormone (ACTH), a basic fibroblast growth factor (bFGF), and a GSK3β inhibitor, in addition to the OCT3/4 transcription stimulating factor, such as LIF. The present invention preferably uses the ACTH and the bFGF in combination with the LIF or the LIF and the CCL2, and the most preferably, the ACTH, the bFGF, and the GSK3β inhibitor are used in combination with the LIF or the LIF and the CCL2.

The GSK3β inhibitor in the present invention means a substance having an inhibitory activity against glycogen synthase kinase 3β (GSK3β). Examples of the GSK3β inhibitor include AR-A014418 (N-(4-methoxybenzyl)-N'-(5-nitro-1,3-thiazol-2-yl)urea), CHIR99021 (6-[[2-[[4-(2,4-dichlorophenyl)-5-(5-methyl-1H-imidazole-2-yl)-2-pyrimidinyl]amino]ethyl]amino]-3-pyridinecarbonitrile), CHIR98014 (N-6-[2-[[4-(2,4-Dichlorophenyl)-5-(1H-imidazol-1-yl)-2-pyrimidinyl]amino]ethyl]-3-nitro-2,6-pyridinediamine), SB415286 (3-[(3-chloro-4-hydroxy phenyl)-amino]-4-(2-nitrophenyl)-1H-pyrrole-2,5-dione), SB216763 (3-(2,4-dichlorophenyl)-4-(1-methyl-1H-indole-3-yl)-1H-pyrrole-2,5-dione), BIO (6-bromoindirubin-3-oxime), and valproic acid, but the GSK3β inhibitor is not limited to them.

The animals from which the OCT3/4 transcription stimulating factor (such as LIF, CCL2, and IL-6), the ACTH, the bFGF, and the GSK3β inhibitor are derived are not especially limited, and the OCT3/4 transcription stimulating factor, the ACTH, the bFGF, and the GSK3β inhibitor derived from any animal, such as a human, a mouse, a rat, a rabbit, a sheep, a pig, and cattle, are usable. Additionally, all of the OCT3/4 transcription stimulating factor, the ACTH, the bFGF, and the GSK3β inhibitor may be recombinants. Commercially available reagents are usable for all of them. All of the OCT3/4 transcription stimulating factor, the ACTH, the bFGF, and the GSK3β inhibitor used in the present invention are preferably materials derived from a human and also preferably recombinants.

The contents of the OCT3/4 transcription stimulating factor (such as LIF, CCL2, and IL-6), the ACTH, the bFGF, and the GSK3β inhibitor in the medium in the step (b) are not especially limited, and can be appropriately set according to the kind of used cells, the culture conditions, and the like.

The content of LIF in the medium is not specifically limited, but, for example, 0.01 to 5000 ng/mL, preferably 0.1 to 1000 ng/mL, and more preferably 1 to 100 ng/mL. Additionally, although not especially limited, assuming that 1/20 of an amount of providing a degree of proliferation 50% of the maximum proliferation is one unit in a cell proliferation promoting assay using a mouse ES cell line (a D3 line), the content of the LIF in the medium is, for example, 10 to 10000 units/ml, preferably 50 to 8000 units/ml, and more preferably 100 to 5000 units/ml. Note that with the use of the LIF by FUJIFILM Wako Pure Chemical (product number: 129-05601), the amount can be diluted by 500 to 2000 times of the amount of medium.

Although the content of the CCL2 in the medium is not specifically limited, for example, 0.1 to 5000 ng/mL, preferably 1 to 1000 ng/mL, and more preferably 10 to 500 ng/mL, 10 to 400 ng/mL, 10 to 300 ng/mL, 50 to 500 ng/mL, 50 to 400 ng/mL, and 50 to 300 ng/mL.

Although the content of the ACTH in the medium is not specifically limited, for example, 0.1 to 200 µmol/L, preferably 1 to 100 µmol/L, and more preferably 5 to 50 µmol/L.

Although the content of the bFGF in the medium is not specifically limited, for example, 0.01 to 5 nmol/L, preferably 0.05 to 3 nmol/L, and more preferably 0.1 to 1 nmol/L.

Although the content of the GSK3β inhibitor in the medium is not specifically limited, for example, 0.1 to 50 µM, preferably 0.5 to 30 µM, and more preferably 1 to 10 µM.

A medium composition used in cell culture in the step (b) can be appropriately set according to the kind of used cells, the culture conditions, and the like. Basically, as long as the medium can maintain the reprogrammed cells, the medium is not specifically limited. For example, although the base medium is not specifically limited, an Eagle medium (such as BM, MEM, and DMEM), a McCoy medium (such as McCoy5A and McCoy7A), a Ham medium (such as F10 and F12), a 199 medium, a RPMI1640 medium, an NCTC medium (such as NCTC109 and NCTC135), and a medium for stem cells can be used. Additionally, various kinds of base media can be mixed to be used as necessary.

To the base medium described above, in addition to the OCT3/4 transcription stimulating factor (such as LIF, CCL2, and IL-6), the ACTH, the bFGF, and the GSK3β inhibitor, an additive preferred to maintain the stem cells, such as serum (such as fetal calf serum and human serum), serum substitute (such as KSR), amino acid (such as alanine, arginine, cystine, and histidine), vitamin (such as vitamin B7 and vitamin B12), an antibiotic (such as amphotericin B and kanamycin), an adhesion factor (such as type I collagen, gelatin, and fibronectin), a growth factor (such as EGF, PDGF, and TGF-α), a cytokine (such as IL-2, IL-3, IL-4, IL-5, and IL-6), a hormone (such as insulin, glucagon, and progesterone), fatty acid (such as oleic acid, arachidonic acid, and linolenic acid), adenine, guanosine, hypoxanthine, thymidine, and cholesterol can be added.

At the step (b), the medium already prepared as the medium for stem cells can be used. For example, StemSure (registered trademark) by FUJIFILM Wako Pure Chemical, a hPSC medium Δ, an ADSC-4 medium by KOHJIN BIO, StemFit by REPROCELL, TeSR series by STEMCELL Technologies, and the like can be used, but the medium is not especially limited to them. The commercially available medium for stem cells may contain the OCT3/4 transcription stimulating factor, the ACTH, the bFGF, or the GSK3β inhibitor. Note that in the step (b), the medium need not be always changed from the step (a). The medium used in the step (a) can be used as is, one or more selected from the group consisting of the LIF, the ACTH, the bFGF, and the GSK3β inhibitor can be contained in this medium, and the step (b) can be performed.

At the step (b), the conditions for culturing the cells are not especially limited. For example, the culture temperature can be 30 to 40°C, preferably 35 to 39°C, and more preferably 36 to 38°C. Additionally, the CO₂ concentration is, for example, 1 to 10%, preferably 1.5 to 8%, and more preferably 2 to 5%.

The culture period of cells in the step (b) is also not especially limited and can be appropriately set according to the kind of used somatic cells, the culture conditions, and the like. The culture period of cells in the step (b) is, for example, 1 day or more, and the specific culture period is 1 to 10 days. Although the preferred culture period is also not especially limited, for example, 2 to 10 days, preferably 3 days or more, and more preferably 3 to 9 days.

In the present invention, as the resultant of the culture of the step (b), a further change in form of the cells is observed in some cases. For example, as illustrated in Fig. 2, a state in which the cells after culture aggregate to form cell aggregates is possibly observed. The cell aggregate can also be referred to as a clump of cells (spheroid). After confirmation of the change in form of the cells in the state, the culture in the step (b) can be stopped.

In the present invention, as described above, performing the step (b) subsequent to the step (a) is a preferred aspect. Although not bound by a specific theory, in a case where an agent that damages a DNA itself or a function of the DNA (such as DNA replication and RNA synthesis. Note that this includes a function of an RNA that brings an indirect action (specifically, a sequence of functions that synthesizes protein from the RNA).) is defined as an "agent making a trigger" (an agent that provides an input of reprograming to cells), and an agent used to maintain the reprogrammed cells (such as LIF and ACTH) is defined as an "agent required to progress initialization"), it is considered that the order of giving the "agent required to progress initialization" to the cells after giving the "agent making a trigger" is important, rather than simultaneously giving the "agent making a trigger" and the "agent required to progress initialization" to the cells. The used concentration of the "agent making a trigger" is a concentration to the extent of not affecting existence of cells.

### <Passage of Cells>

In the present invention, in the cultures at the step (a) and the step (b), passage of the cells is possible. The passage operation of cells is not especially limited, and can be performed appropriately according to the kind of used cells, the culture conditions, and the like. For example, when the cells in a confluent state can be confirmed by microscope observation or the like, the medium is removed from a culture container, a buffer solution, such as PBS (-), is added to the culture container to clean the surfaces of the cells, and then a protease, such as trypsin, is added to this container to ensure collecting the cells. The collected cells are added in a new medium, and passage of the cells can be performed through a sequence of operations.

The number of times of passage of cells can be set to 1 to 10 times, but is not especially limited, and can be appropriately set according to the kind of used somatic cells, the culture conditions, and the like. Although not specifically limited, in the present invention, the number of passages of cells is preferably one to five times, more preferably one to three times, further preferably one or two times, and the most preferably one time.

The present invention is a method for creating reprogramed cells from somatic cells without gene introduction that includes: a step (a) of culturing the somatic cells in a medium containing a histone deacetylase inhibitor; and a step (b) of culturing the cells cultured in the step (a) in a medium containing an OCT3/4 transcription stimulating factor to create the reprogrammed cells.

The reprogrammed cells are multipotent stem cells that are allowed to be differentiated into three germ layers. The multipotent stem cells are possibly pluripotent.

### <Multipotent/Pluripotent Stem Cells>

The multipotent stem cells in this Description mean cells having an autoreproduction ability of performing division and proliferation with an undifferentiated state maintained and an ability of ensuring differentiation into cells in a plurality of lineages. Preferably, the multipotent stem cells of the present invention have multipotency/pluripotency of ensuring differentiation into cell lines belonging to three germ layers (endoderm, mesoderm, and ectoderm).

The pluripotent stem cells in this Description mean cells having an autoreproduction ability of performing division and proliferation with an undifferentiated state maintained and multipotency/pluripotency of ensuring differentiation into all cell lines belonging to three germ layers (endoderm, mesoderm, and ectoderm). The pluripotent stem cells have a teratoma forming ability and a chimera forming ability.

The "multipotent" and the "pluripotent" are not always clearly discriminated. In this Description, since a possibility of having the teratoma forming ability (tumorigenicity) is low, the reprogrammed cells of the present invention are described as "multipotent," but in a sense generally used, the case of these cells being "pluripotent" is not eliminated.

Confirmation that the cells obtained by the method of the present invention are the highly undifferentiated cells can be appropriately performed using the method publicly known to those skilled in the art. As one confirmation method, for example, alkaline phosphatase activation is examined. The alkaline phosphatase activation can be appropriately examined using, for example, a commercially available kit for alkaline phosphatase stain.

Whether the stem cells are the multipotent/pluripotent stem cells or not can be determined by, for example, confirmation of expression of, for example, Nanog, Klf4, Oct4, Sox2, c-Myc, Lin28, TRA-1-60, and SSEA (such as SSEA-4 and SSEA-1) as marker genes of the multipotent/pluripotent stem cells. The expression of marker gene can be confirmed using the method publicly known to those skilled in the art, such as RT-PCR, and as long as the confirmation is performed at a protein level, the device and the method are publicly known, such as an antibody specific to various kinds of markers and a FACS, can be used.

The multipotency/pluripotency can be confirmed by examining the differentiation into the three germ layers, the endoderm, the mesoderm, and the ectoderm. The differentiation into the three germ layers can be confirmed by examining markers of the respective germ layers. Examples of the endoderm marker include Sox17, CXCR4, HNP-3β, FoxA2, AFP, GATA-4, PDX-1, and Nkx2.1, examples of the mesoderm marker include MSX1, α-SMA, Obt2, and Brachyury, and examples of the ectoderm marker include Pax6, MAP2, Nestin, Otx2, TP63, and SOX2. The various kinds of markers can be confirmed using the method publicly known to those skilled in the art, similarly to the confirmation of the marker of multipotent/pluripotent stem cells.

For example, cells are injected into under a skin of a mouse to form teratoma and the differentiated tissue is analyzed, thus ensuring evaluating the teratoma forming ability. The teratoma includes the differentiated tissue derived from the three germ layers, the endoderm, the mesoderm, and the ectoderm. For example, cells are implanted into a blastocyst and whether a chimera animal is formed from this blastocyst is examined, thus ensuring confirming chimera forming ability. Both of the teratoma forming ability and the chimera forming ability can be examined using the method publicly known to those skilled in the art.

Differentiation potency can be examined in vitro using the differentiation induction method publicly known to those skilled in the art. Differentiation into various kinds of cells may be performed using a commercially available kit for differentiation induction and the like.

### <Method for Producing Cells>

The present invention also provides a method for producing cells using multipotent/pluripotent stem cells obtained by the method described above as another aspect. Specifically, the present invention provides a method for producing cells that includes the following steps (i) and (ii): a step (i) of creating the reprogrammed cells from the somatic cells by the method described above; and a step (ii) of performing differentiation induction on the reprogrammed cells into the aimed cells.

The step (i) can be performed in combination with the step (a) or in combination of the step (b) with this. Additionally, the differentiation induction of the cells in the step (ii) can be appropriately performed using the method publicly known to those skilled in the art according to the kind of the aimed cells.

Although the cells obtained by the production method of the present invention are not specifically limited, examples of which include adipose cells, nerve cells, muscle cells (such as cardiac muscle cells, smooth muscle cells, and skeletal muscle cells), skin cells, epithelial cells, endothelial cells, blood cells (such as neutrophil, eosinophil, basophil, monocyte, and lymphocyte), hepatocytes, nephrocytes, lung cells, pancreatic cells, breast cells, and hair cells.

### <Kit and Composition>

As another aspect, the present invention provides the kit to reprogram cells including a medium containing an HDAC inhibitor. Additionally, as yet another aspect, the present invention provides the composition to reprogram cells including a medium containing an HDAC inhibitor. The HDAC inhibitor and the medium containing it are as described in the step (a) in the method of the present invention described above.

A kit of the present invention, for example, is constituted by the following two reagents:
a reagent A) a reagent for constituting a medium not containing 2-mercaptoethanol; and a reagent B) a reagent for constituting a medium containing LIF. The media and components for the reagent A and the reagent B may be separately packed and prepared at time of use, or the respective components may be contained in the media.
Additionally, the medium containing the reagent A may further contain CCL2 and/or IL-6 as the OCT3/4 transcription stimulating factor.

In addition to the LIF, the CCL2, and the IL-6, the reagent B can contain one or more selected from the group consisting of the ACTH, the bFGF, and the GSK3β inhibitor. The media used for the reagent A and the reagent B are as described in the steps (a) and (b) in the method of the present invention described above.

### <Pharmaceutical Composition, Cosmetic Composition>

The present invention can provide the cells reprogrammed by the method of the present invention or cell masses thereof. Although the cells of the present invention are not especially limited, for example, since the cells are obtained using the HDAC inhibitor, one of the features is not including exogenous gene introduction regarding Oct3/4, Sox2, Klf4, c-Myc, and the like. Additionally, although the cell masses (cell aggregates) of the cells reprogrammed by the method of the present invention are not especially limited, for example, as shown in a working example (Fig. 2B) described below, one of morphological features is that a boundary of the cell mass is unclear, and the inside is ununiform.

The cells reprogrammed by the method of the present invention, the differentiated cells of these cells (the cells produced by differentiation of the cells reprogrammed by the method of the present invention), or an extracted component or a secretory component of these cells (the component extracted from the cells reprogrammed by the method of the present invention or the component secreted by these cells) can be used as an active constituent of the pharmaceutical composition or the cosmetic composition. That is, as another aspect, the present invention provides the pharmaceutical composition or the cosmetic composition containing the cells reprogrammed by the method of the present invention described above, the differentiated cells of these cells, or the extracted component or the secretory component of these cells. Additionally, as another aspect, the present invention provides a method for producing a pharmaceutical composition or a cosmetic composition including a step for combining the cells reprogrammed by the method of the present invention described above, the differentiated cells of these cells, or the extracted component or the secretory component of these cells.

Although the component extracted from the cells reprogrammed by the method of the present invention or the component secreted by these cells is not specifically limited, examples of the component include a gene-related substance, such as miRNA, DNA, or RNA, protein, a cytokine, an extracellular vesicle (including an exosome), and miRNA, DNA, RNA, and protein contained in the extracellular vesicle. One kind of them may be used or two or more kinds of them may be combined.

Although an application of the pharmaceutical composition is not specifically limited, examples of the application include treatments for dysosmia, cerebral infarction, diabetes, neuropathy, cancer, and a liver disease. In a case where the cells reprogrammed by the method of the present invention are used, these cells themselves or the cells differentiated from these cells can be used for cell transplantation.

The pharmaceutical composition or the cosmetic composition of the present invention can contain a pharmacologically acceptable carrier according to the form. Examples of the pharmacologically acceptable carrier include an excipient, a binder, an emulsifier, a tonicifier (isotonizing agent), a buffer, a solubilizing agent, a preservative, a stabilizing agent, an antioxidant agent, a colorant, a coagulant, and a coating agent, but the carrier is not limited to them.

As another aspect, the present invention also can provide the method for treating any of the above-described diseases and use for treatment of any of the above-described diseases for the above-described applications. That is, as another aspect, the present invention provides a method for treating any of the above-described diseases using the cells reprogrammed by the method of the present invention described above, the differentiated cells of these cells, or the extracted component or the secretory component of these cells. Additionally, as another aspect, the present invention provides use of the cells reprogrammed by the method of the present invention described above, the differentiated cells of these cells, or the extracted component or the secretory component of these cells for treating any of the above-described diseases.

### [Working Examples]

Although the present invention will be further specifically described in the following working examples, they are merely examples, and do not limit the scope of the present invention by any means.

### [Working Example 1]

### 1. Reprogramming Somatic Cells

Normal human skin fibroblasts (adult) (Takara Bio) were seeded to a spheroid plate, and after 24 to 48 hours, medium exchange was performed on a D-MEM (Low Glucose) medium to which 2-mercaptoethanol diluted by 1/100000 (142 µM) was added. Then, after three to four days, medium exchange was performed again on the same D-MEM medium.

As described above, after the human skin fibroblasts were cultured for one week, the change in form of the cells was confirmed (Fig. 1). After the change in form of the cells was confirmed, medium exchange was performed on the medium in which LIF (wako, used by 1:1000), ACTH (wako, 10 µmol/L), bFGF (wako, 0.34 nmol/L), and AR-A014418 (Merck, 3 µM) were added to ADSC-4 (KOHJIN BIO). Then, after three or four days, the medium exchange was performed again on the same medium.

Culturing the cells for one week in the ADSC-4 medium containing the LIF and the like as described above further aggregated the cells, thus forming spheroids (Fig. 2).

### 2. Alkaline Phosphatase Reaction

The alkaline phosphatase reaction of the cells finally obtained by the above-described culture was examined. The stain of alkaline phosphatase was performed in accordance with the operating manual attached to the kit using Alkaline Phosphatase Live Stain (Invitrogen). Specifically, the cells were cultured for 30 minutes in a medium in which a staining fluid (AP Live Stain) attached to the kit diluted by 500 times was added in the D-MEM. Afterwards, the medium was exchanged and cleaning was performed for five minutes × two times. After the cleaning, when the product was observed with a fluorescence microscope using a GFP filter for determination of presence/absence of the stain, the stain was observed (Fig. 3).

### 3. Examination of Various Kinds of Conditions

### (1) 2-Mercaptoethanol Concentration

The processes similar to those of the above-described items 1 and 2 were performed to examine the concentration of 2-mercaptoethanol. Specifically, except that the concentration of 2-mercaptoethanol was set to 0.142 µM, 1.42 µM, 14.2 µM, 142 µM, 1.42 mM, 14.2 mM, and 142 mM, the operations same as those in the items 1 and 2 were performed. Consequently, the spheroid formation of the cells and the alkaline phosphatase stain were observed in the 2-mercaptoethanol at the concentration of 0.142 µM to 1.42 mM, and the alkaline phosphatase stain was especially strongly observed in the 2-mercaptoethanol at the concentration of 14.2 µM to 1.42 mM. Note that when the 2-mercaptoethanol was 14.2 mM or more, extinction of the cells was observed.

### (2) Used Medium

Similarly to the item 1, after performing the culture in the D-MEM medium containing the 2-mercaptoethanol (142 µM), LIF (wako, used by 1:1000), ACTH (wako, 10 µmol/L), bFGF (wako, 0.34 nmol/L), and AR-A014418 (Merck, 3 µM) were added in this medium and further the cells were cultured for one week. As a result, both of spheroid formation of the cells and the alkaline phosphatase stain were observed. Additionally, when the culture only in the D-MEM medium containing the 2-mercaptoethanol (142 µM) was performed as in the item 1, spheroid formation of the cells and the weak alkaline phosphatase stain were observed. However, in only the culture in the medium in which LIF (wako, used by 1:1000), ACTH (wako, 10 µmol/L), bFGF (wako, 0.34 nmol/L), and AR-A014418 (Merck, 3 µM) were added to the ADSC-4 medium, neither spheroid formation of the cells nor the alkaline phosphatase stain was observed.

### (3) Factors of LIF and ACTH

In the item 1, when (i) only the LIF, (ii) only the ACTH, or (iii) only the LIF and the ACTH were added to the ADSC-4 medium for culture, spheroid formation of the cells and the alkaline phosphatase stain were observed under any conditions. In the conditions (i) and (iii), the alkaline phosphatase stain was especially observed strongly.

### 4. Expression of Undifferentiated Markers

Whether gene expression had been generated was examined on Nanog, Klf4, Oct4, and Sox2 as the undifferentiated markers. The RNA was collected from the cells using a RNeasy Mini Kit (QIAGEN). PCR was performed on the above-described various kinds of undifferentiated markers using the method described in Cell. 2007 Nov 30; 131(5): 861-72.

Consequently, bands were able to be confirmed in all of Nanog, Klf4, Oct4, and Sox2 of the tested cultured cells around the targeted band sizes (Nanog: 406 bp, Klf4: 396 bp, Oct4: 143 bp, and Sox2: 150 bp) (Fig. 4). Note that absence of a band was able to be confirmed in negative control.

### 5. Differentiation Induction to Adipose Cells

The differentiation induction test to the adipose cells was conducted on the cells obtained in the item 1 using a commercially available cell differentiation kit. The kit used for the test was Mesenchymal Stem Cell Adipogenic Differentiation Medium 2 (PromoCell, product code C-28016). The culture solution of cells was exchanged for the culture solution attached to the kit, and thereafter the cells were cultured in accordance with the operating manual attached to the kit.

Fat droplets observed in the cells on which the differentiation induction was performed were stained using LipiDye (Funakoshi). The stain was performed in accordance with the operating manual attached to the kit and live cell imaging was performed. Specifically, the cells were cultured for two hours in the medium in which the staining fluid attached to the kit was added in the D-MEM such that a final concentration became 1 µM. Afterwards, the medium was exchanged and cleaning was performed for five minutes × two times. After the cleaning, when the product was observed with a fluorescence microscope using a GFP filter for determination of presence/absence of the stain, the stain was observed (Fig. 5).

### 6. Differentiation Induction to Nerve Cells

The differentiation induction test to the nerve cells was conducted on the cells obtained in the item 1 using a commercially available cell differentiation kit. The kit used for the test was Mesenchymal Stem Cell Neurogenic Differentiation Medium (PromoCell, product code C-28015). The culture solution of cells was exchanged for the culture solution attached to the kit, and thereafter the cells were cultured in accordance with the operating manual attached to the kit.

Immunostaining was performed on the cells on which the differentiation induction had been performed using an anti-neurofilament antibody (abcam, ab7255, Anti-68kDa Neurofilament/NF-L antibody [DA2]) as an antibody against the nerve cells (Fig. 6).

### 7. Differentiation Induction to Hepatocytes

The differentiation induction test to the hepatocytes was conducted on the cells obtained in the item 1. The cells were cultured in a 0.8-µM hexachlorophene/D-MEM medium in accordance with the method described in Scientific Reports (2015) 5:16169.

A PAS stain method, a Western Blotting method, and an indocyanine green test were performed on the cells on which the differentiation induction had been performed to confirm the properties of hepatocytes. The PAS stain was performed using a commercially available PAS stain kit (MUTO PURE CHEMICALS) in accordance with the operating manual attached to the kit. In Western Blotting, a cell dissolution was prepared using CellLytic M (Sigma Aldrich) in accordance with the operating manual attached to the product. After sodium dodecyl sulfate-polyacrylamide gel electrophoresis (SDS-PAGE) was performed on the prepared cell dissolution in accordance with the method of Laemmli, the cell dissolution was transcribed to a PVDF membrane by semi-dry method, and Western-blot analysis was performed. As a detected primary antibody, an anti-albumin rabbit polyclonal antibody (4929: Cell Signaling Technology) and an anti-β-Actin mouse monoclonal antibody (A5441: Sigma Aldrich) were used. As a secondary antibody, a HRP labelled antibody against IgG derived from an animal creating the detected primary antibody was used. To detect a signal, a luminescence imaging device LAS4000 (FUJIFILM) was used. The indocyanine green test was conducted in compliance with the method described in Cloning Stem Cells (2007) Spring; 9(1):51-62, and observation was performed after 1 hour and 20 hours after starting the stain (Fig. 8).

### 8. Differentiation Induction to Cartilage Cells

The differentiation induction test to the cartilage cells was conducted on the cells obtained in the item 1 using a commercially available cell differentiation kit. The kit used for the test was Mesenchymal Stem Cell Chondrogenic Differentiation Medium (PromoCell, product code C-28012). The culture solution of cells was exchanged for the culture solution attached to the kit, and thereafter the cells were cultured in accordance with the operating manual attached to the kit.

HE stain, alcian blue stain, immunostaining (an S-100 antibody, a GFAP antibody, a CD34 antibody, an SMA antibody, and a Vimentin antibody) were performed on the cells on which the differentiation induction had been performed, and protein expressed in the cells was evaluated (Fig. 11). When randomly selected fibrocartilage and hyaline cartilage in a human clinical specimen were compared and examined, the protein expression similar to the fibrocartilage was recognized, and it was apparent that the cartilage cell obtained by differentiation induction was similar to the fibrocartilage.

### 9. Differentiation Induction to Osteoblasts

In accordance with the item 1, after culture in the D-MEM medium containing 2-mercaptoethanol, the differentiation induction test to the cartilage cells was conducted on the cells cultured in the ADSC-4 medium containing the LIF, the ACTH, and the bFGF using a commercially available osteoblast differentiation medium. The medium used for the test is Mesenchymal Stem Cell Osteogenic Differentiation Medium (PromoCell, product code C-28013).

Alkaline phosphatase stain was performed on the cells after the differentiation induction and it was confirmed that the cells were positive in alkaline phosphatase (Fig. 12).

### [Working Example 2]

### 1. Effect by CCL2

The CCL2 was added to the LIF and an influence given to reprogramming was verified. In accordance with Working Example 1, while medium exchange was performed on a normal human skin fibroblast (adult) (the Takara Bio) in a D-MEM medium containing 2-mercaptoethanol (142 µM) for three to four days, culture was performed for seven days. Next, culture was performed for additional seven days in a medium in which (i) only LIF, (ii) LIF + CCL2 (10 ng/mL), or (iii) LIF + CCL2 (100 ng/mL) were added to an ADSC-4 medium. Note that the LIF was used by 1:1000.

The change in form (a form in which a projecting part is slightly depressed and slightly rounded from a form of a spindle shape is formed) was confirmed in the cells after culture in (i) to (iii) (Fig. 13). Especially, addition of the CCL2 made cell aggregation remarkable, contours between the cells became clarified, and divergence of cell adhesion was recognized (Fig. 13D).

### 2. Differentiation Induction to Adipose Cells

The differentiation induction test to the adipose cells was conducted on the cells cultured in the media containing (ii) LIF + CCL2 (10 ng/mL) and (iii) LIF and CCL2 (10 ng/mL) in accordance with the item 5 in Working Example 1 using a commercially available cell differentiation kit.

Cells on the 25th day after differentiation induction were stained using LipiDye (Funakoshi) (Figs. 14 and 15). Although fat droplets were confirmed in all of the cells, it was recognized that the number of formed fat droplets and the sizes of fat droplets were increased in the cells in (iii) compared with (ii).

Furthermore, a difference in differentiation induction to the adipose cells was examined including a case of providing a period during which culture was performed only in the medium between a period during which culture was performed in the presence of 2-mercaptoethanol (a step A) and a period during which culture was performed in the presence of LIF (a step B).
The step A (3 to 9 days) → culture period only in the medium (0 to 14 days) → step B (3 to 9 days) → adipose differentiation induction (14 days)

First, each of the step A and the step B was performed for 3 days, and the culture periods in the media were set to 0 days, 3 days, 7 days, and 10 days.
- Fat droplets were generated by differentiation induction after the step A: 3 days → the step B: 3 days.
- The fat droplets were generated by differentiation induction after the step A: 3 days → the medium: 3 days → the step B: 3 days.
- The fat droplets were generated by differentiation induction after the step A: 3 days → the medium: 7 days → the step B: 3 days.
- The fat droplets were generated by differentiation induction after the step A: 3 days → the medium: 10 days → the step B: 3 days.

When the culture period in the medium was 0 days, 3 days, and 7 days, after differentiation induction, small but many fat droplets were generated on the 14th day, and the fat droplets grew to large sizes and the number of fat droplets increased on the 21st day. In the case of the medium period in the medium of 10 days, after differentiation induction, the number of fat droplets was considerably small on the 14th day.

The culture period in the medium was set to 0 days, and the culture periods in the step A and the step B were changed.
- The fat droplets were generated by differentiation induction after the step A: 5 days and the step B: 7 days.
- The fat droplets were generated by differentiation induction after the step A: 7 days and the step B: 7 days.
- The fat droplets were generated by differentiation induction after the step A: 9 days and the step B: 7 days.
- On the 14th day after differentiation induction, it seemed that the smaller the density was, the larger the number of fat droplets was. However, the fat droplets grew to large sizes irrespective of the density after about 21 days, and the number of fat droplets increased.

From the results described above, it was able to be confirmed that, as the culture period, three days were sufficient for each of the step A and the step B.

### INDUSTRIAL APPLICABILITY

The cell reprogramming method provided by the present invention is effective to study and development of a tissue regeneration technique, and can be used in the field of regeneration medicine, for example, creation of an organ for regeneration medicine. Additionally, since cells can be changed to another organ, for example, in the field other than medical treatment of a human, skin cells of cattle can be changed to muscle cells for food for humans.

All published materials, patents, and patent applications cited in this Description are incorporated herein by reference.

## Claims

1. A method for creating reprogramed cells from somatic cells without gene introduction, the method comprising:
a step (a) of culturing the somatic cells in a medium containing a histone deacetylase inhibitor; and
a step (b) of culturing cells cultured in the step (a) in a medium containing an OCT3/4 transcription stimulating factor to create the reprogrammed cells.

2. The method according to claim 1, wherein
the OCT3/4 transcription stimulating factor is any one or more selected from the group consisting of LIF, CCL2, and IL-6.

3. The method according to claim 1 or 2, wherein
the histone deacetylase inhibitor is 2-mercaptoethanol.

4. A method for creating reprogramed cells from somatic cells without gene introduction, the method comprising:
a step (a) of culturing somatic cells in a medium containing 2-mercaptoethanol; and
a step (b) of culturing cells cultured in the step (a) in a medium containing LIF to create the reprogrammed cells.

5. The method according to any one of claims 1 to 4, wherein
the medium in the step (a) contains 0.1 µM to 2 mM of 2-mercaptoethanol.

6. The method according to claim 4 or 5, wherein
the medium in the step (b) further contains CCL2.

7. The method according to any one of claims 1 to 6, wherein
the medium in the step (b) further contains one or more selected from ACTH, bFGF, and a GSK3β inhibitor.

8. The method according to any one of claims 1 to 7, wherein
the medium in the step (a) does not contain the OCT3/4 transcription stimulating factor.

9. The method according to any one of claims 1 to 8, wherein
the reprogrammed cells are multipotent stem cells that are allowed to be differentiated into three germ layers.

10. The method according to any one of claims 1 to 9, wherein
the somatic cells are fibroblasts.

11. The method according to any one of claims 1 to 10, wherein
the somatic cells are human skin fibroblasts.

12. A kit for reprogramming somatic cells containing the following reagent A and reagent B:
the reagent A) a reagent for constituting a medium containing 2-mercaptoethanol; and
the reagent B) a reagent for constituting a medium containing LIF.

13. The kit according to claim 12, wherein
the reagent B further contains one or more selected from CCL2, ACTH, bFGF, and a GSK3β inhibitor as a component.

14. A method for producing aimed cells from somatic cells, the method comprising:
a step (i) of creating the reprogrammed cells from the somatic cells by the method according to any one of claims 1 to 11; and
a step (ii) of performing differentiation induction on the reprogrammed cells to the aimed cells.

15. A method for producing a cell preparation comprising:
a step (i) of creating the reprogrammed cells from the somatic cells by the method according to any one of claims 1 to 11;
a step (ii) of performing differentiation induction on the reprogrammed cells to aimed cells; and
a step (iii) of preparing a cell preparation containing a pharmacologically acceptable carrier with differentiation inducted cells.
